# EUROPÄISCHE PATENTSCHRIFT

(11) **EP 2 845 907 B1**
(45) Veröffentlichungstag und Bekanntmachung des Hinweises auf die Patenterteilung: **03.01.2018**
(21) Anmeldenummer: 14181243.8
(22) Anmeldetag: 18.08.2014
(51) Int. Cl.: C12Q 1/24, G01N 33/569, C12M 1/26

(54) **Handmessgerät und Verfahren zum Nachweis von Schimmelpilzbefall in Innenräumen**
Handheld measuring device and method for the detection of mildew infestation in indoor spaces
Appareil de mesure portatif et procédé de détection de formation de moisissure dans les espaces intérieurs

(30) Priorität: 06.09.2013 DE 102013217822
(43) Veröffentlichungstag der Anmeldung: 11.03.2015
(73) Patentinhaber: ROBERT BOSCH GMBH, 70442 Stuttgart (DE)
(72) Erfinder: Luckert, Katrin, 71229 Leonberg (DE)

(56) Entgegenhaltungen:
- WO-A1-2008/156415
- WO-A1-2012/089417
- WO-A2-2007/092302
- M CARLSON ET AL: "An automated, handheld biosensor for aflatoxin", BIOSENSORS AND BIOELECTRONICS, Bd. 14, Nr. 10-11, 1. Januar 2000 (2000-01-01), Seiten 841-848, XP055164190, ISSN: 0956-5663, DOI: 10.1016/S0956-5663(99)00057-3

## Beschreibung

Die vorliegende Erfindung betrifft ein Verfahren zum Nachweis von verdeckten Schimmelschäden in Innenräumen. Die vorliegende Erfindung betrifft ferner ein Handmessgerät zur Ausführung des Nachweisverfahrens.

### Stand der Technik

Schimmelpilzbefall in Innenräumen und daraus resultierende Schimmelschäden treten auch in Neubauten auf und können mit auf Schimmelsporen zurückgehenden gesundheitlichen Folgen verbunden sein. Um Innenräume auf Pilzbefall zu testen werden verschiedenen Nachweismethoden verwendet. So können Schimmelsporen durch Filtration oder Impaktion aus der Raumluft oder Staubsammlungen gesammelt and anschließend für beispielsweise 7 Tage im Labor kultiviert werden, woraufhin ein Nachweis anhand der Kultur erfolgt. Jedoch muss selbst bei offensichtlichem Pilzbefall eine große Menge an Sporen gesammelt und über mehrere Tage kultiviert werden. Ebenfalls können mikroskopische Untersuchungen an Partikelsammlungen durchgeführt werden, wobei auch nicht kultivierbare Sporen erfasst werden. Neben diesen klassischen Labortests sind sogenannte Do-it-yourself Schnelltests bekannt.

Vorrichtungen und Verfahren zur Untersuchung auf Schimmelpilzbefall sind im Stand der Technik bekannt. Aus dem Dokument DE 10 2010 064 251 A1 ist beispielsweise ein Handmessgerät und ein Verfahren zum Nachweis von verdeckten Schimmelschäden in Innenräumen bekannt.

Eine mögliche Ausgestaltung eines Handmessgerätes zur Detektion von Aflatoxinen wird beispielsweise durch M Carlson el al. in "An automated, handheld biosensor for aflatoxin",Biosensors and Bioelectronics, Bd. 14, Nr. 10-11, 1. Januar 2000 (2000-01-01), Seiten 841-848, XP05516419O beschreiben.

Eine weitere Möglichkeit zum Aufbau eines Handmessgerätes und eines Verfahrens zum Nachweis von Schimmelpilzbefall in Innenräumen wird zudem in der WO 2012/089417 A1 offenbart.

Nach wie vor ist jedoch die Probenvorbereitung ein wichtiger Teil des gesamten Analysevorgangs einer Realprobe, insbesondere wenn nur geringe Mengen eines Analyten in der Luft zu bestimmen sind.

### Offenbarung der Erfindung

Gegenstand der vorliegenden Erfindung ist ein Handmessgerät zum Nachweis von verdeckten Schimmelschäden in Innenräumen, gemäß Anspruch 1.

Gegenstand der vorliegenden Erfindung ist auch ein Verfahren zum Nachweis von verdeckten Schimmelschäden in Innenräumen mit dem zuvor beschriebenen Handmessgerät, gemäß Anspruch 2.

Ein Kompartiment kann im Sinne der vorliegenden Erfindung insbesondere ein weitgehend abgegrenzter Raum sein. Dies kann durch eine Teilung der Puffereinheit zur Verfügung gestellt werden. Die Puffereinheit weist wenigstens ein erstes und ein zweites Kompartiment auf, kann jedoch auch drei oder mehr Kompartimente aufweisen.

Kern der Erfindung ist eine der Messung vorangehende Aufkonzentrierung der Probe. Die Probe, insbesondere angesaugte Innenraumluft, wird in einer Sammeleinheit gesammelt. Anschließend wird die Probe in einem Puffer aufgenommen. Vor der immunologischen Messung erfolgt in der Puffereinheit erfindungsgemäß eine Probenaufkonzentrierung. Hierzu ist die Puffereinheit des Handmessgerätes kompartimentiert.

Vorteile der Erfindung liegen darin, dass die anschließende Messung direkt durchgeführt werden kann. Hierdurch werden langwierige Sammelprozesse verkürzt. Die Probenmenge wird insbesondere durch die Stärke des Luftstroms und die Dauer der Probensammlung geregelt. Die Aufkonzentrierung während der Probennahme kann einerseits den Zielanalyten, Schimmelpilzsporen, spezifisch anreichern, andererseits kann hierdurch eine Vorreinigung durchgeführt werden. So können Nicht-Analyte abgetrennt werden. Mögliche Störeffekte können hierdurch reduziert werden. Insbesondere durch eine Affinitätsreinigung kann die Probe in höherer Konzentration sowie einem speziellen Messpuffer zur Messung vorgelegt werden. Insgesamt wird durch die Aufkonzentration im Rahmen der Probenvorbereitung ein wichtiger Schritt für die anschließende Analyse zur Verfügung gestellt, der den Nachweis von Schimmelsporen wesentlich erleichtert. Dies ist insbesondere für die Umsetzung eines Schnelltests wesentlich. Die Erfindung ermöglicht einen semi-quantitativen Schnelltest zur schnellen vor-Ort Detektion von Schimmelsporen aus Luft- und Staubproben einzusetzen. Die hierzu notwendige Probenmenge kann schnell und spezifisch aus niedrig konzentrierten Medien wie Luft angereichert werden. Dies stellt eine Grundlage für ein portables Messgerät unabhängig von klassischer Laboranalytik zur Verfügung.

Die Probe wird zunächst in einer Sammeleinheit des Handmessgerätes aufgenommen. Dazu können Bestandteile von Baumaterialien wie Tapeten, Putzstäube oder Ähnliches auf einen Träger innerhalb des Gerätes manuell appliziert werden. Bevorzugt wird jedoch eine Sammeleinheit nach dem Impaktions- und/oder Filtrationsverfahren. Dementsprechend weist die Sammeleinheit vorzugsweise eine Prallplatte und/oder einen Filter auf. Bei der Prallplatte handelt es sich um ein Trägerplättchen, das mit einem Klebstoff zur Adhäsion der Schimmelpilzbestandteile beschichtet ist. Für die Filtration der Luft werden spezielle Filtermembranen eingesetzt, die sich zur Filtration von Schimmelpilzen bzw. deren Bestandteilen wie Sporen eignen. Dabei werden die sich in der Luft befindenden Partikel mit einer Größe ab etwa 0,5 µm abgeschieden und auf der Filtermembran gesammelt. Um einen Luftstrom zu erzeugen, verfügt die Sammeleinheit vorzugsweise über ein Luftansaugmodul, z.B. in Form einer Pumpe oder eines Ventilators. Der Luftansaugstrom kann ca. 100 l/min betragen. Auch Stäube können auf diese Art aus der Luft abgeschieden und gesammelt werden. Um Kontaminationen zu vermeiden, wird die eigentliche Sammeleinheit, d.h. die Prallplatte bzw. der Filter als Einweg-Komponente in das Gerät eingelegt.

Die gesammelte Probe wird in eine Pufferlösung aufgenommen. Der Sammeleinheit nachgeordnet ist entsprechend eine Puffereinheit. Die gesammelte Probe wird in ein erstes Kompartiment der Puffereinheit überführt. Im Puffer gelöst liegen Bindermoleküle für den Analyten, die Schimmelpilzsporen, vor. Nach der Zugabe werden vorhandene Schimmelpilzsporen von den Bindemolekülen gebunden und liegen als Komplex in Lösung vor. Die Lösung wird in das zweite Kompartiment der Puffereinheit überführt. Dort wird der Komplex von Bindemolekül und Schimmelpilzspore immobilisiert. Das zweite Kompartiment der Puffereinheit eine Festphase zur Immobilisierung des Bindemolekül-Analyt-Komplexes auf. Eine Festphase kann im Sinne der vorliegenden Erfindung insbesondere eine nichtwässrige Matrix sein, an die das Bindemolekül haften kann. Die Festphase kann eine Reinigungssäule oder eine diskontinuierliche Phase sein. Die Festphase umfasst bevorzugt eine carboxylierte Oberfläche. An dieser carboxylierten Overfläche ist ein weiteres Bindungsmolekül immobilisiert, das spezifisch das erste Bindungsmolekül und somit den Komplex aus ersten Bindungsmolekül und Analyt an der Festphase bindet.

Werden Antikörper, welche sich spezifisch gegen Oberflächenmarker von Schimmelsporen wie β-Glukane richten, als erstes Bindemolekül verwendet (Primärantikörper), so kann das zweite Bindnungsmolekül ein Antikörper (Sekundärantikörper), der sich spezies-spezifisch sich gegen den Primärantikörper richtet. Der Sekundärantikörper muss bereits immobilisiert vorliegen. Dies kann beispielsweise über Kopplungsreagenzien wie s-NHS und EDC (N-hydroxysulfosuccinimide und carbodiimide) als Amidbindung kovalent an einer carboxylierten Oberfläche erfolgen. Nicht gebundene Moleküle in der Probe können durch Zugabe einer Spüllösung entfernt werden. Anschließend kann die über das

Bindemolekül gebundene Schimmelspore mittels eines weiteren Puffers eluiert und wieder in Lösung gebracht werden.

Erfindungsgemäß weist die Puffereinheit des Handmessgeräts wenigstens drei Kartuschen für Puffer auf. Die Pufferkartuschen enthalten verschiedene Puffer. Beispielsweise kann eine erste Pufferkartusche einen Puffer enthalten, der Bindemoleküle in Lösung enthält, eine zweite Pufferkartusche kann einen Puffer ohne Bindemoleküle enthalten. Dieser kann zum Spülen der Festphase bzw. zum Entfernen nicht gebundener Bestandteile verwendet werden. Eine dritte Pufferkartusche kann einen so genannten Elutionspuffer enthalten, der zum Ablösen der gebundenen Analyte dient. Es kann auch vorgesehen sein, dass die Bindemolekülen getrennt zugegeben werden. Hierfür kann eine weitere Kartusche vorgesehen sein. Auch die Zugabe weiterer Testkomponenten mit Hilfe zusätzlicher Kartuschen oder einer separat anzuschließenden Fluidik zur Bevorratung und dosierten Zugabe entsprechender Komponenten ist möglich.

In einer gesonderten Ausführungsform ist die Sammeleinheit und die Puffereinheit zu einer Einheit zusammengefasst, so dass das Sammeln der Probe und die Aufnahme in den Puffer zeitgleich erfolgt. Die Kartuschen mit Pufferlösung können ebenfalls als Einweg-Komponente ausgeführt sein und zusammen oder getrennt mit der Sammeleinheit in das Messgerät eingesetzt werden. Damit die gesammelte Probe in eine Pufferlösung aufgenommen wird, muß die Pufferkartusche zunächst geöffnet werden. Dies erfolgt entweder automatisch, sobald die Probennahme abgeschlossen ist, oder wird vom Anwender explizit ausgelöst. Entsprechend kann die Zugabe der weiteren Puffer aus den jeweiligen Katuschen als automatisierte Abfolge erfolgen, oder jeweils vom Anwender ausgelöst werden. Um die Aufnahme und die Durchmischung der Probe in der Pufferlösung zu verbessern, kann eine separate Durchmischung erfolgen, die z.B. mit mechanischen Hilfsmitteln erfolgt.

Die Probe wird aus der Puffereinheit in die immunologische Testeinheit überführt. Je nach Ausführung des immunologischen Tests, kann die Testeinheit Komponenten zur Reagenzienzugabe, vorzugsweise eine Reagenzienkartusche, oder Lateral-Flow-Testkomponenten aufweisen. Beim Lateral-Flow-Test erfolgt eine unmittelbare Bestimmung der Analyten. Die immunologische Testeinheit wird vorzugsweise als Einmaltest verwendet, so dass zu jeder Messung ein Austausch der Testkomponenten in Form eines Teststreifens und/oder einer Reagenzienkartusche erfolgt. Alternativ kann die Testeinheit auch mehrfach verwendet werden, so dass ein Austausch nach jeder Einzelmessung entfällt. Insgesamt können die Prallplatte und/oder der Filter, die Pufferkartuschen, die Reagenzienkartusche oder die Lateral-Flow-Testkomponenten in unterschiedlicher Kombination oder einzeln als Einweg-Komponenten ausgeführt sein und entsprechend einzeln oder in Kombination in das Handmessgerät eingelegt bzw. angeschlossen werden.

Für den immunologischen Test kommen unterschiedliche Transduktionsprinzipien, z.B. in Form von optischen oder elektrischen Signalen, in Frage. Vorzugsweise erfolgt das Auswerten des immunologischen Tests durch elektronische oder optische Sensoren. Daher verfügt die Auswertungseinheit vorzugsweise über ein fotometrisches oder elektronisches Transduktionsmodul. Die Funktionsweise hängt von dem jeweiligen Messprinzip ab. So ist z.B. eine fotometrische Analyse der Probe, aber auch eine Impedanzmessung oder eine fluorimetrische Messungmöglich. Generell ermöglicht das Gerät über einen oder mehrere Immuntests die parallele Bestimmung von mehreren Antigenen oder Haptenen. Hierzu stehen z.B. multianalytfähige Lateral-Flow-Tests (LFT) zur Verfügung. Markierte Detektionsantikörper können entweder bereits in der Pufferlösung vorgelegt werden oder sich immobilisiert auf der Aufnahmefläche der Testeinheit befinden. Sind entsprechende Antigene oder Haptene in der Probe vorhanden, binden die dadurch entstehenden Antigen-Antikörper-Konjugate an die entsprechenden Detektionsbereiche, die anschließend über die Auswertungseinheit optisch oder anderweitig ausgelesen werden. Es können solche Antigene oder Haptene bestimmt werden, die charakteristisch für Schimmelpilze im Allgemeinen sind wie z.B. Ergosterol oder beta-1,3-Glucan. Das Vorliegen dieser Substanzen weist darauf hin, dass in der Probe Schimmelpilze vorhanden sind. Desweiteren können solche Antigene oder Haptene bestimmt werden, die charakteristisch für spezielle Schimmelpilzarten sind, z.B bestimmte Oberflächenantigene von Schimmelpilzsporen. Solche differenzierten Analysen lassen Rückschlüsse zu, ob die Pilze mit großer Wahrscheinlichkeit von Außen eingetragen wurden oder ob sie aus einem Innenraumschaden stammen. Als für Innenraumbefälle charakteristischen Arten können z.B. die folgenden Pilzsporen gemessen werden: *Acremonium spp., Aspergillus penicillioides, Aspergillus restrictus, Aspergillus versicolor, Aureobasidium pullulans, Chaetomium spp., Phialophora spp., Scopulariopsis brevicaulis, Scopulariopsis fusca, Stachybotrys chartarum, Tritirachium (Engyodontium) album* oder *Trichoderma spp..*

Die Anzeige des Testergebnisses kann in unterschiedlicher Form erfolgen. Vorzugsweise erfolgt die Anzeige des Testergebnisses über ein Kontrollfeld. Bei einer fotometrischen Auswertung kann das Ergebnis unmittelbar anhand einer oder mehrerer charakteristischer Farbänderungen oder Verfärbungen, die gegebenenfalls im Abgleich mit einer Farbskala eine qualitative oder halbquantitative Aussage ermöglichen, abgelesen werden. So ist eine schnelle Aussage in Form von "nicht nachweisbar", "geringe Belastung" oder "hohe Belastung" möglich. Alternativ führt das Gerät intern eine optische oder elektrische Auswertung durch, welche als Ergebnis qualtitativ, halbquantitativ oder quantitativ durch eine elektronische Anzeige, z.B. über ein Display oder einzelne LEDs, angezeigt wird. Darüberhinaus verfügt das Gerät vorzugsweise über eine Kontrollanzeige, welche die korrekte Ausführung des Test bestätigt oder gegebenenfalls auf mögliche Fehlerquellen hinweist. Das Gerät kann sowohl mit Akkumulatoren als auch mit herkömmlichen Batterien betrieben werden.

Kern des Verfahrens ist eine der immunologischen Messung vorangehende Aufkonzentrierung der Probe. Vorteile des Verfahrens liegen darin, dass zum einen die Zielanalyten aus der Luft selektiv gebunden werden können und damit eine Aufkonzentrierung der Zielanalyten erreicht werden kann, sowie des weiteren darin, dass störende Bestandteile aus der Probe entfernt werden können, so dass Querempfindlichkeitsreaktionen reduziert werden. Insbesondere durch eine Affinitätsreinigung kann die Probe in höherer Konzentration sowie einem speziellen Messpuffer zur Messung vorgelegt werden. Insgesamt wird durch die Aufkonzentration im Rahmen der Probenvorbereitung eine wesentliche Erleichterung für die anschließende Analyse zum Nachweis von Schimmelsporen zur Verfügung gestellt. Die Probenmenge kann schnell und spezifisch aus niedrig konzentrierten Medien wie Luft angereichert werden. Somit ermöglicht das erfindungsgemäße Verfahren innerhalb eines Messsystems die Nachweisbarkeit eines Analyten, der ansonsten nicht in ausreichender Konzentration für eine Messung vorliegt.

In einem ersten Schritt wird insbesondere Luft angesaugt und an einer Sammeleinrichtung gesammelt. Dabei kann das Sammeln der Probe nach dem Impaktions- oder Filtrationsverfahren, d.h. mit Hilfe von adhäsiv beschichteten Prallplatten oder unter Einsatz von geeigneten Filtermembranen, durchgeführt werden. Anschließend wird die Probe in einer Pufferlösung aufgenommen. Das Sammeln der Probe und die Aufnahme der Probe in den Puffer bzw. die Pufferlösung können zeitgleich erfolgen, so dass die Probe noch während des Sammelvorgangs im Puffer suspendiert wird. Zur besseren Verteilung der Probe kann nach der Aufnahme der Probe in den Puffer ein Durchmischen der Lösung erfolgen. Dabei kann die gepufferte Probe z.B. durch Vibration oder Rühren mechanisch durchmischt werden.

Ein Puffer kann im Sinne der vorliegenden Erfindung insbesondere eine gepufferte Lösung sein, die sich Veränderungen des pH-Wertes durch Wirkung eines konjugierten Säure-Base-Paares widersetzt. Vorzugsweise stellt ein Puffer physiologische Bedingungen für den Analyten bereit.

Erfindungsgemäß wird in der in einen Puffer aufgenommenen Probe vor der immunologischen Testung der oder die Analyte aufkonzentriert. Erfindungsgemäß wird das Aufkonzentrieren mittels Analyt-spezifischer Bindemoleküle durchgeführt. Im Rahmen einer weiteren Ausgestaltung können die Bindemoleküle Antikörper oder Komplexbildner sein, die spezifisch den oder die Analyten binden. Dies können im Falle von Schimmelsporen als

Analyten Antikörper sein, welche sich spezifisch gegen Oberflächenmarker von Schimmelsporen wie β-Glukane richten. Der Begriff "Antikörper" umfasst im Sinne der vorliegenden Erfindung insbesondere monoklonale Antikörper, polyklonale Antikörper, wie auch multispezifische Antikörper.

Das Aufkonzentrieren umfasst die folgenden Schritte:
- Binden des oder der Analyte an Bindemoleküle,
- Immobilisieren des Bindemolekül-Analyt-Komplexes,
- Entfernen nicht gebundener Bestandteile,
- Ablösen der gebundenen Analyte.

Die Bindemoleküle für den Analyten können in dem Puffer gelöst vorliegen, in welchem die Probe aufgenommen wird. Alternativ können Bindermoleküle wie Antikörper oder Komplexbildner, die spezifisch den Zielanalyten binden, zur Probenlösung gegeben werden. Nach der Zugabe werden vorhandene Pilzsporen von den Bindemolekülen gebunden und liegen als Komplex oder Konjugat in Lösung vor. Die Lösung wird dann in ein weiteres Kompartiment der Puffereinheit überführt, wo der Bindemolekül-Analyt-Komplex insbesondere Pilzspore-Antikörper-Konjugate immobilisiert wird. Der Komplex aus Analyt und Primär-Antikörper kann über bereits an der Festphase gebundene Sekundärantikörper, die über Kopplungsreagenzien wie s-NHS und EDC (N-hydroxysulfosuccinimide und carbodiimide) über eine Amidbindung kovalent an einer carboxylierten Oberfläche immobilisiert sind, gebunden werden. In einem weiteren Schritt werden nicht gebundene Moleküle und Bestandteile in der Probe entfernt. Dies kann durch Zugabe einer Spüllösung erfolgen. Die Spüllösung weist dabei insbesondere die Eigenschaften auf, physiologische Bedingungen einzuhalten, um die Antigen-Antikörper Bindung nicht zu lösen und den Zielanalyten, die Schimmelspore, nicht wegzuspülen. Hierzu ist insbesondere eine Pufferlösung geeignet, die der Pufferlösung entspricht, in der die Probe aufgenommen wurde, die jedoch keine Bindemoleküle enthält. Abschließend wird der gebundenen Analyt wieder von dem Bindemolekül abgelöst. Vorzugsweise werden an Antikörper gebundene Schimmelsporen unter Verwendung eines so genannten Elutionspuffers wieder in Lösung gebracht. Unter einem Elutionspuffer werden generell Pufferlösungen verstanden, die einen Ananlyten von einem immobilisierten Bindepartner Iösen. Dazu sind die Puffer so zu wählen, dass die Antigen-Antikörper Bindung zwischen Schimmelspore und Primär-Antikörper gelöst wird. Dies kann beispielsweise über eine Variation des pH-Wertes durchgeführt werden, der die Wechselwirkung zwischen zwei Proteinen beeinflusst. Im Gegensatz dazu bleibt der kovalent gebundene SekundärAntikörper an der Festphase gebunden. Durch die Zugabe eines Elutionspuffers werden die Analyte von den Bindemolekülen getrennt und liegen wieder in Lösung vor. Diese Lösung wird zur weiteren Messung in eine Messkammer oder immunologische Testeinheit überführt.

Im Rahmen einer Ausgestaltung können die Bindemoleküle Antikörper (Primärantikörper) sein, welche sich gegen Oberflächenmarker von Schimmelsporen wie β-Glukane richten. Werden Antigene wie β-Glukane bestimmt, die charakteristisch für Schimmelpilze im Allgemeinen sind, erfolgt eine allgemeine Anreicherung aller in der Probe enthaltenen Schimmelsporen. Im Rahmen einer weiteren Ausgestaltung können die Bindemoleküle spezies-spezifische Antikörper gegen Schimmelsporen sein, beispielsweise Antikörper gegen Oberflächenantigene, die charakteristisch für spezielle Schimmelpilzarten sind. In diesem Fall können ganz bestimmte Schimmelspezies für den Nachweis angereichert oder selektiert werden. Somit kann eine Antikörper-basierte Vorreinigung neben einer allgemeinen Anreicherung aller in der Probe enthaltenen Schimmelsporen über den Einsatz von spezies-spezifischer Antikörper aus einem allgemeinen Test auf Schimmelsporen zu einem qualitativen Test auf bestimmte Schimmelspezies ausgebaut werden. Insbesondere kann somit die Wahl der Antikörper darüber entscheiden, ob ein Summensignal über alle in der Probe enthaltenen Schimmelsporen oder nur ein spezies-spezifisches Signal einer Schimmel-Spezies gemessen wird. Die Auswahl des Sekundär-Antikörpers ist abhängig von der Herkunft des PrimärAntikörpers. Dies bedeutet, dass bei Verwendung eines Primärantikörpers aus der Maus ein gegen Mäuse-Antikörper gerichteter Sekundärantikörper eingesetzt werden muss.

Zur Messung kann die Ananlytkonzentration über definierte Volumenzugaben an Pufferlösung angepasst werden. Die Analyse der flüssigen Probe wird mittels immunologischen Tests durchgeführt und visualisiert. Mit der immunologischen Testung in Schritt c) kann auf mindestens eine Komponente ausgewählt aus der Gruppe von: Ergosterol, β-1,3-Glucan, und für Schimmelpilze artspezifische Marker, getestet werden. Im Rahmen einer Ausgestaltung kann das Testergebnisses aus Schritt d) einem Summensignal über mehrere in der Probe enthaltene Schimmelsporen oder einem spezies-spezifischen Signal einer Schimmel-Spezies entspricht. Um in einem Innenraum gemessenen Werte mit Referenzwerten abgleichen zu können, kann das Gerät auch im Außenbereich angewendet werden, um so Schimmelpilze, die typischerwesie im Innenbreich vorkommen, von denen im Außenbereich abzugrenzen.

### Beispiele und Zeichnungen

Weitere Vorteile und vorteilhafte Ausgestaltungen der erfindungsgemäßen Gegenstände werden durch die Zeichnungen veranschaulicht und in der nachfolgenden Beschreibung erläutert. Dabei ist zu beachten, dass die Beispiele und Zeichnungen nur beschreibenden Charakter haben und nicht dazu gedacht sind, die Erfindung in irgendeiner Form einzuschränken. Es zeigen
- Fig. 1: eine schematische Ansicht einer Ausführungsform eines erfindungsgemäßen Handmessgerätes; und
- Fig. 2: eine schematische Wiedergabe der Verfahrensschritte des Aufkonzentrierens mittels Analyt-spezifischer Bindemoleküle.

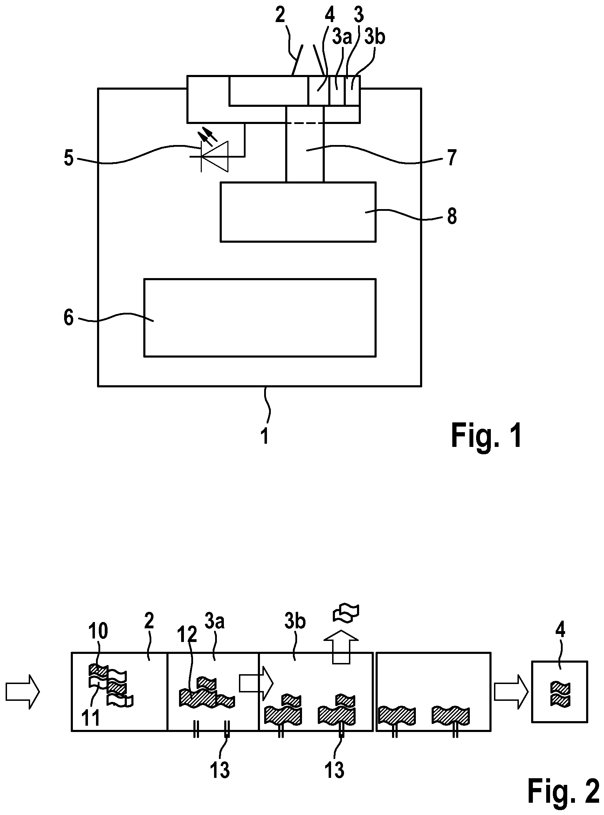

Die Figur 1 zeigt schematisch eine Ausführungsform des erfindungsgemäßen Handmessgerätes 1. Das Gerät umfasst eine Sammeleinheit 2 für Luft und Staub. Die Sammeleinheit 2 umfasst eine Pumpe oder einen Lüfter als Ansaugmodul 8 sowie einen Luft- bzw. Staubkanal 7. Kern der Sammeleinheit 2 ist ein Impaktor oder Filter, auf der die in der Luft befindlichen Sporen der Schimmelpilze abgeschieden und gesammelt werden. Der Filter oder Impaktor ist als Einweg-Komponente ausgeführt. Die Puffereinheit 3 weist ein erstes Kompartiment 3a und ein zweites Kompartiment 3b auf. Die Pufferlösungen befinden sich in Einweg-Kartuschen und sind innerhalb der Puffereinheit 3 angeordnet. Innerhalb des ersten Kompartiments 3a ist eine Kartusche enthaltend einen Puffer, der Antikörper gegen β-Glukane von Schimmelsporen enthält, angeordnet. Die Schimmelsporen aus Luft und Staub werden nach Abschluss des Sammelvorgangs in diese Pufferlösung aufgenommen. Die Lösung enthaltend die an Antikörper gebundenen Schimmelsporen wird in das zweite, das Bindungs-Kompartiment 3b überführt. Innerhalb des zweiten Kompartiments 3b sind zwei weitere Kartuschen enthaltend einen Puffer ohne Antikörper und einen Elutionspuffer, angeordnet. Weiterhin enthält das zweite Kompartiment 3b eine Festphase mit über eine carboxylierte Oberfläche gebundenen Sekundärantikörper zur Immobilisierung der Antikörper-Schimmelpilz-Komplexe. Nach Spülen der Festphase mit Puffer ohne Bindemoleküle zur Entfernung nicht gebundener Bestandteile, werden die gebundenen Schimmelsporen durch Zugabe des Elutionspuffers abgelöst. Der die nun aufkonzentrierten Schimmelsporen enthaltende Elutionspuffer wird in die immunologische Testeinheit 4 überführt. Innerhalb der Testeinheit 4 ist ein Einweg-Immunoassay für die Schimmelpilzsporen angeordnet. Die Auswertungseinheit 5 umfasst eine optische Auswerteoptik. Neben der notwendigen Auswertungselektronik weist das Handmessgerät 1 außerdem ein Kontrollfeld 6 bzw. Display auf, mit dem die Ergebnisse der Messung angezeigt werden.

### Ausführungsbeispiel

Das Handmessgerät zur Ausführung des Nachweisverfahrens beinhaltet eine Akku-betriebene Filtrationsvorrichtung mit einem Luftansaugstrom von ca. 100 l/min. Die sich im Luftstrom befindlichen Partikel > 0.5 µm werden durch eine geeignete Filtrationsmembran zurückgehalten und gesammelt. Der Filtrationsaufsatz besteht aus einer Ansaugdüse sowie einer Filtrationsmembran und ist zusammen mit drei Pufferkartuschen und dem immunologischen Schnelltest als kunststoffbasierte Einwegkomponente integriert. Vor der Luftpartikelsammlung wird diese Wegwerfeinheit in das Handmessgerät eingelegt. Die Luftpartikelsammlung erfolgt über wenige Minuten.

Die Figur 2 zeigt eine schematische Wiedergabe der Verfahrensschritte des Aufkonzentrierens mittels Analyt-spezifischer Bindemoleküle. Die eingesogenen Partikel werden in einem Sammelraum 2 des Handmessgeräts gesammelt. Im Anschluss wird per Knopfdruck die erste Kartusche mit einer Pufferlösung aufgebrochen. Die Pufferlösung bietet physiologische Bedingungen für Schimmelsporen. Im Puffer gelöst liegen als Bindemoleküle Primär-Antikörper vor, welche sich spezifisch gegen β-Glukane als Oberflächenmarker von Schimmelsporen richten. Die Pufferlösung gelangt dadurch auf die Filtrationsmembran und die gesammelten Analyte 10 sowie Nicht-Analyte 11 werden in dem ersten Kompartiment 3a der Puffereinheit in der Lösung suspendiert. Vorhandene Schimmelsporen werden von den Primär-Antikörpern 12 spezifisch gebunden und liegen als Komplex in Lösung vor. Die Lösung wird mittels Knopfdruck in das Bindungs-Kompartiment 3b überführt.

Im Kompartiment 3b liegen bereits über Kopplungsreagenzien wie s-NHS und EDC (N-hydroxysulfosuccinimide und carbodiimide) als Amidbindung kovalent an eine carboxylierten Oberfläche gebundene Sekundärantikörper 13 vor. Per Knopfdruck wird nun die zweite Kartusche mit Pufferlösung aufgebrochen, die frei von Bindemolekülen ist. Diese Pufferlösung hält ebenfalls physiologische Bedingungen ein, um die Antigen-Antikörper Bindung nicht zu lösen und die Schimmelsporen nicht wegzuspülen. Beispielsweise entspricht diese Pufferlösung in ihrer Zusammensetzung der ersten Pufferlösung, enthält jedoch keine Primär-Antikörper. Durch die Zugabe werden nicht gebundene Bestandteile 11 entfernt, während die Antigen-Antikörper-Komplexe immobilisiert an der Festphase verbleiben. Nach dem Spülen wird durch einen weiteren Knopfdruck eine dritte Kartusche mit Elutionspuffer aufgebrochen. Der Elutionspuffer unterscheidet sich beispielsweise im pH-Wert von den Puffern, die zur Aufnahme der Probe und zum Spülen der Festphase verwendet werden. Durch die Zugabe des Elutionspuffers wird die Antigen-Antikörper Bindung getrennt und die Schimmelsporen werden von den Bindemolekülen abgelöst. Die an der Festphase kovalent immobilisierten Sekundärantikörper bleiben gebunden. Die Lösung enthält nun eine vorgereinigte Probe, in der der Zielanalyt durch die Antikörperbindung gezielt aufkonzentriert wurde. Über eine definierte Volumenzugabe des Elutionspuffers kann die Ananlytkonzentration in der Lösung zur Messung angepasst werden. Dies ist ebenfalls manuell über Knopfruck steuerbar.

Durch einen weiteren Knopfdruck gelangt die Probe, in der die Schimmelsporen nun wieder in Lösung vorliegen, in die in die immunologische Testeinheit 4 zur Aufnahmefläche des immunologischen Schnelltests auf Basis eines multianalytfähigen Lateral Flow Tests (LFT). Die markierten Detektionsantikörper befinden sich immobilisiert auf der Aufnahmefläche. Bei Vorhandensein der zu detektierenden Analyten binden Antigen-Antikörper-Konjugate an die designierten Detektionsbereiche der Auswertungseinheit. Die optisch ausgelesenen Messwerte der verschiedenen Analyten, die dem Ergosterol-/ beta-1,3-Glucan-Gehalt entsprechen, werden auf einen Mikrokontroller übertragen, der auf Basis der gespeicherten Kalibrationsdaten sowie der angesaugten Luftmenge die Messwerte in die jeweilige Schimmelpilzsporenzahl pro Kubikmeter angesaugter Luft umrechnet. Um ein besserers quantitatives Ergebnis zu erhalten und den korrekten Testablauf zu prüfen, kann zusätzlich eine Referenzfläche der Filtermembran ausgewertet werden.

Abhängig von der Wahl des Antikörpers während des Aufkonzentrierens wird ein Summensignal über alle in der Probe enthaltenen Schimmelsporen oder ein spezies-spezifisches Signal einer bestimmtem Schimmel-Spezies gemessen.

Die Ausgabe des Ergebnisses erfolgt auf einem Kontrollfeld bzw. Display. Darüber erhält der Anwender die Information ob Schimmelpilze gefunden wurden, d.h. Ergosterol- bzw. beta-1,3-Glucan nachgewiesen werden konnte, und ob innenraumspezifische Arten gefunden wurden. Die Messwerte können entweder direkt als Sporenzahl pro Kubikmeter gesammelter Luft angegeben werden, oder bereits eine Bewertung durchlaufen. Dazu ist ein Bewertungsschema im Messgerät hinterlegt, mit welchem die Messwerte abgeglichen werden. Nach Ablauf der Messung oder vor der nächsten Messung wird die Einwegkomponente aus dem Handgerät entnommen.

## Patentansprüche

1. Handmessgerät (1) zum Nachweis von verdeckten Schimmelschäden in Innenräumen, aufweisend folgende Komponenten:
- mindestens eine Sammeleinheit (2) zur Aufnahme mindestens einer Innenraumprobe,
- mindestens eine Puffereinheit (3), welche wenigstens drei Kartuschen für einen Puffer aufweist,
- mindestens eine immunologische Testeinheit (4),
- eine Auswertungseinheit (5), und
- ein Kontrollfeld (6),
wobei die Puffereinheit (3) ein erstes Kompartiment (3a) mit Bindemolekülen für einen Analyten und ein zweites Kompartiment (3b) mit einer Festphase zur Immobilisierung eines Bindemolekül-Analyt-Komplexes aufweist.

2. Verfahren zum Nachweis von verdeckten Schimmelschäden in Innenräumen, umfassend folgende Verfahrensschritte:
a) Sammeln einer Innenraumprobe,
b) Aufnehmen der Probe in einem Puffer,
c) immunologische Testung der gepufferten Probe
d) Auswerten des immunologischen Tests, und
e) Anzeigen des Testergebnisses aus Schritt d),
wobei in der in Schritt b) in einen Puffer aufgenommenen Probe vor der immunologischen Testung in Schritt c) ein oder mehrere Analyt(e) mittels Analyt-spezifischer Antikörper oder Komplexbildner aufkonzentriert wird, wobei das Aufkonzentrieren die folgenden Schritte umfasst:
- Binden des oder der Analyte an Bindemoleküle,
- Immobilisieren des Bindemolekül-Analyt-Komplexes,
- Entfernen nicht gebundener Bestandteile,
- Ablösen der gebundenen Analyte, wobei ein Handmessgerät nach Anspruch 1 verwendet wird.

3. Verfahren nach Anspruch 2, **dadurch gekennzeichnet, dass** die Bindemoleküle Antikörper sind, welche sich gegen Oberflächenmarker von Schimmelsporen wie β-Glukane richten.

4. Verfahren nach Anspruch 2 oder 3, **dadurch gekennzeichnet, dass** die Bindemoleküle spezies-spezifische Antikörper gegen Schimmelsporen sind.

5. Verfahren nach Anspruch 2 bis 4, **dadurch gekennzeichnet, dass** das Testergebnis aus Schritt d) einem Summensignal über mehrere in der Probe enthaltene Schimmelsporen oder einem spezies-spezifischen Signal einer Schimmel-Spezies entspricht.

## Claims

1. Handheld measuring device (1) for detecting concealed mould damage in interior spaces, having the following components:
- at least one collection unit (2) for receiving at least one interior space sample,
- at least one buffer unit (3) which has at least three cartridges for a buffer,
- at least one immunological testing unit (4),
- an evaluation unit (5), and
- a control field (6),
wherein the buffer unit (3) has a first compartment (3a) with binding molecules for an analyte and a second compartment (3b) with a solid phase for immobilizing a binding molecule analyte complex.

2. Method for detecting concealed mould damage in interior spaces, comprising the following method steps:
a) collecting an interior space sample,
b) receiving the sample in a buffer,
c) immunologically testing the buffered sample,
d) evaluating the immunological test, and
e) displaying the test result from step d),
wherein, prior to the immunological test in step c), the concentration of one or more analyte(s) is increased by means of analyte-specific antibodies or complexing agents in the sample received in a buffer in step b), wherein increasing the concentration comprises the following steps:
- binding the analyte or analytes to the binding molecule,
- immobilizing the binding molecule analyte complex,
- removing non-bound constituents,
- releasing the bound analytes, wherein use is made of a handheld measuring device according to Claim 1.

3. Method according to Claim 2, **characterized in that** the binding molecules are antibodies which are directed against surface markers of mould spores such as β-glucans.

4. Method according to Claim 2 or 3, **characterized in that** the binding molecules are species-specific antibodies against mould spores.

5. Method according to Claims 2 to 4, **characterized in that** the test result from step d) corresponds to an aggregate signal over a plurality of mould spores contained in the sample or a species-specific signal for one mould species.

## Revendications

1. Appareil de mesure portatif (1) permettant de détecter des ravages de moisissures cachés dans des espaces intérieurs, présentant les composants suivants:
- au moins une unité de collecte (2) pour recueillir au moins un échantillon d'espace intérieur,
- au moins une unité tampon (3), qui présente au moins trois cartouches pour un tampon,
- au moins une unité de test immunologique (4),
- une unité d'analyse (5), et
- un champ de contrôle (6),
dans lequel l'unité tampon (3) présente un premier compartiment (3a) avec des molécules de liaison pour un analyte et un deuxième compartiment (3b) avec une phase solide pour l'immobilisation d'un complexe molécule de liaison-analyte.

2. Procédé pour détecter des ravages de moisissures cachés dans des espaces intérieurs, comprenant les étapes de procédé suivantes:
a) collecte d'un échantillon d'espace intérieur,
b) placement de l'échantillon dans un tampon,
c) test immunologique de l'échantillon placé en tampon,
d) analyse du test immunologique, et
e) affichage du résultat du test de l'étape d),
dans lequel on concentre dans l'échantillon placé dans un tampon à l'étape b), avant le test immunologique de l'étape c), un ou plusieurs analyte (s) au moyen d'anticorps ou d'agents complexants spécifiques des analytes, dans lequel la concentration comprend les étapes suivantes:
- lier le ou les analyte(s) à des molécules de liaison,
- immobiliser le complexe molécule de liaison-analyte,
- éliminer les composants non liés,
- séparer les analytes liés, dans lequel on utilise un appareil de mesure portatif selon la revendication 1.

3. Procédé selon la revendication 2, **caractérisé en ce que** les molécules de liaison sont des anticorps, qui agissent à l'encontre de marqueurs de surface de spores de moisissures comme des β-glucanes.

4. Procédé selon la revendication 2 ou 3, **caractérisé en ce que** les molécules de liaison sont des anticorps spécifiques d'espèces contre des spores de moisissures.

5. Procédé selon une des revendications 2 à 4, **caractérisé en ce que** le résultat du test de l'étape d) correspond à un signal de somme portant sur plusieurs spores de moisissures contenues dans l'échantillon ou à un signal spécifique d'espèce d'une espèce de moisissure.
